# EUROPEAN PATENT SPECIFICATION

(11) **EP 4 426 252 B1**
(45) Date of publication and mention of the grant of the patent: **25.06.2025**
(21) Application number: 22790368.9
(22) Date of filing: 29.09.2022
(51) Int. Cl.: A61F 13/00, A61F 13/02, A61M 1/00

(54) **NEGATIVE PRESSURE WOUND THERAPY DRESSING WITH ISOLATED SUPER ABSORBENT**
UNTERDRUCKWUNDTHERAPIEVERBAND MIT ISOLIERTEM SUPERABSORBENS
PANSEMENT DE THÉRAPIE DE PLAIE À PRESSION NÉGATIVE AVEC SUPER-ABSORBANT ISOLÉ

(30) Priority: 05.11.2021 US 202163263617 P
(43) Date of publication of application: 11.09.2024
(73) Proprietor: KCI Manufacturing Unlimited Company, Athlone, Co. Westmeath, N37XF22 (IE)
(72) Inventor: MCGREGOR, Andrew J., Saint Paul, Minnesota 55133-3427 (US); HIGLEY, Kevin, Saint Paul, Minnesota 55133-3427 (US); SANDBACK, Tyler J., Saint Paul, Minnesota 55133-3427 (US); MARTINEZ, Diana C., Saint Paul, Minnesota 55133-3427 (US)
(74) Representative: Simmons & Simmons
(86) International application number: PCT/IB2022/059300
(87) International publication number: WO 2023/079382

(56) References cited:
- CN-A- 107 929 833
- US-A1- 2013 131 616
- US-A1- 2016 199 546

## Description

### TECHNICAL FIELD

The invention set forth in the appended claims relates generally to tissue treatment systems and more particularly, but without limitation, to a negative pressure wound therapy dressing including an isolation layer configured to restrict fluid flow to an absorbent material during the application of negative pressure.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative- pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro- deformation of tissue at a wound site. Together, these benefits can increase development of granulation tissue and reduce healing times.

While the clinical benefits of negative-pressure therapy are widely known, improvements to therapy systems, components, and processes may benefit healthcare providers and patients.
US2013/0131616 discloses a negative pressure treatment system for treating a plurality of tissue sites including a valve to prevent flow of fluid to one of the tissue sites.
US2016/0199546 discloses a negative pressure wound dressing including a barrier layer to separate the dressing into an exudate receptable and a contact layer receptacle.
CN107929833 discloses a negative pressure drainage assembly with an anti-backflow drainage device.

### BRIEF SUMMARY

The invention is defined by the independent claim. A selection of optional features is set out in the dependent claims.

### BRIEF DESCRIPTION OF THE DRAWINGS

Figure 1 is a block diagram of an example embodiment of a therapy system that can provide negative pressure treatment in accordance with this specification;
Figure 2 is a cut-away view of an illustrative embodiment of the therapy system of Figure 1 depicting an illustrative example embodiment of a dressing interface and a dressing deployed at a tissue site;
Figure 3 is a cut-away view of the dressing of Figure 2;
Figure 4 is a detail view taken at reference FIG. 4, depicted in Figure 2, illustrating the dressing of Figure 2 positioned proximate to tissue surrounding the tissue site;
Figure 5 is an exploded view of the dressing of Figure 2, depicted with an illustrative example embodiment of a release liner for protecting the dressing prior to application at a tissue site;
Figure 6 is a plan view of an illustrative example embodiment of a base layer depicted in the dressing of Figure 5;
Figure 7A is a perspective view of an illustrative example embodiment of an isolation layer depicted in the dressing of Figure 5 when negative pressure is applied to the dressing and one or more valves associated with the isolation layer are closed;
Figure 7B is a perspective view of an illustrative example embodiment of the isolation layer depicted in the dressing of Figure 5 in a resting state when the one or more valves are open;
Figure 7C is a cut-away view of the isolation layer of Figure 7A, illustrating one or more holes through the isolation layer being closed by the valves;
Figure 7D is a cut-away view of the isolation layer of Figure 7B, illustrating a passage through the holes in the isolation layer when the valves are open;
Figure 8A is a cut-away view of the system illustrating an operative embodiment of the therapy system of Figure 2 delivering negative pressure to the tissue site;
Figure 8B is a cut-away view of the system illustrating an operative embodiment of the system of Figure 2 when negative pressure is not being delivered to the tissue site;
Figure 9A is a perspective view of another illustrative example embodiment of an isolation layer that can be used with the dressing of Figure 5, shown when negative pressure is applied to the dressing;
Figure 9B is a perspective view of the illustrative example embodiment of the isolation layer of FIG. 9A in a resting state without negative pressure being applied;
Figure 9C is a cut-away view of the isolation layer of Figure 9A, illustrating a plurality of misaligned holes through the isolation layer that are sealed when negative pressure is applied;
Figure 9D is a cut-away view of the isolation layer of Figure 9B; illustrating a passage through the holes in the isolation layer when negative pressure is removed;
Figure 10A is a perspective view of another illustrative example embodiment of an isolation layer that can be used with the dressing of Figure 5; and
Figure 10B is a cut-away view of the isolation layer of Figure 10A.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

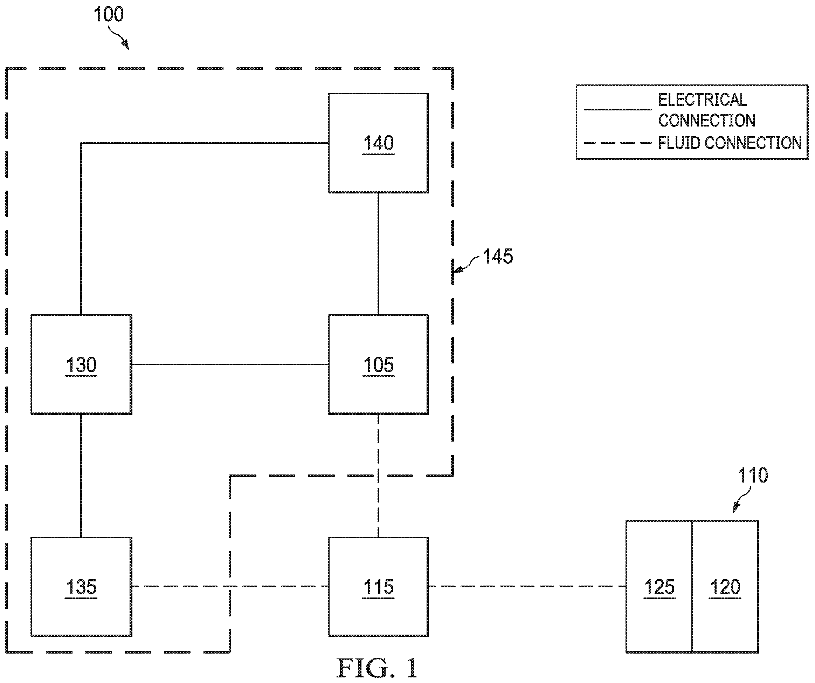

Figure 1 is a block diagram of an example embodiment of a therapy system 100 that can provide negative-pressure therapy to a tissue site in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of Figure 1, the dressing 110 may comprise or consist essentially of a tissue interface 120, a cover 125, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in Figure 1, for example, the therapy system 100 may include a first sensor 135 and a second sensor 140 coupled to the controller 130.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130 and other components into a therapy unit 145.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between - 50 mm Hg (-6.7 kPa) and -300 mm Hg (-39.9 kPa).

The container 115 is representative of a container, a canister, a pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, can be any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may comprise or consist essentially of a manifold. A manifold in this context may comprise or consist essentially of a means for collecting or distributing fluid across the tissue interface 120 under pressure. For example, a manifold may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, a manifold may comprise a plurality of pathways, which can be interconnected to improve distribution or collection of fluids. In some illustrative embodiments, a manifold may comprise or consist essentially of a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

In some embodiments, the tissue interface 120 may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) may be particularly suitable for some types of therapy. The tensile strength of the tissue interface 120 may also vary according to needs of a prescribed therapy. The 25% compression load deflection of the tissue interface 120 may be at least 0.35 pounds per square inch (0.00241 MPa) and the 65% compression load deflection may be at least 0.43 pounds per square inch (0.00296 MPa). In some embodiments, the tensile strength of the tissue interface 120 may be at least 10 pounds per square inch (0.06895 MPa). The tissue interface 120 may have a tear strength of at least 2.5 pounds per inch (0.01724 MPa). In some embodiments, the tissue interface may be foam comprised of polyols such as polyester or polyether, isocyanate such as toluene diisocyanate, and polymerization modifiers such as amines and tin compounds. In some examples, the tissue interface 120 may be reticulated polyurethane foam such as found in GRANUFOAM^{™} dressing or V.A.C. VERAFLO^{™} dressing, both available from Kinetic Concepts, Inc. of San Antonio, Texas.

The thickness of the tissue interface 120 may also vary according to needs of a prescribed therapy. For example, the thickness of the tissue interface may be decreased to reduce tension on peripheral tissue. The thickness of the tissue interface 120 can also affect the conformability of the tissue interface 120. In some embodiments, a thickness in a range of about 5 millimeters to 10 millimeters may be suitable.

The tissue interface 120 may be either hydrophobic or hydrophilic. In an example in which the tissue interface 120 may be hydrophilic, the tissue interface 120 may also wick fluid away from a tissue site, while continuing to distribute negative pressure to the tissue site. The wicking properties of the tissue interface 120 may draw fluid away from a tissue site by capillary flow or other wicking mechanisms. An example of a hydrophilic material that may be suitable is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, the tissue interface 120 may be constructed from bioresorbable materials. Suitable bioresorbable materials may include, without limitation, a polymeric blend of polylactic acid (PLA) and polyglycolic acid (PGA). The polymeric blend may also include, without limitation, polycarbonates, polyfumarates, and capralactones. The tissue interface 120 may further serve as a scaffold for new cell-growth, or a scaffold material may be used in conjunction with the tissue interface 120 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may comprise or consist of, for example, an elastomeric film or membrane that can provide a seal adequate to maintain a negative pressure at a tissue site for a given negative-pressure source. The cover 125 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 125 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some embodiments, the cover 125 may comprise INSPIRE 2301 having an MVTR (upright cup technique) of 2600 g/m²/24 hours and a thickness of about 30 microns.

An attachment device may be used to attach the cover 125 to an attachment surface, such as undamaged epidermis, a gasket, or another cover. The attachment device may take many forms. For example, an attachment device may be a medically acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

The process of reducing pressure may be described illustratively herein as "delivering," "distributing," or "generating" negative pressure, for example. In general, exudate and other fluid flow toward lower pressure along a fluid path. Thus, the term "downstream" typically implies a location in a fluid path relatively closer to a source of negative pressure or further away from a source of positive pressure. Conversely, the term "upstream" implies a location relatively further away from a source of negative pressure or closer to a source of positive pressure. However, the fluid path may also be reversed in some applications, such as by substituting a positive-pressure source for a negative-pressure source; and therefore, these descriptive terms should not be construed as limiting.

Negative pressure applied to the tissue site through the tissue interface 120 in the sealed therapeutic environment can induce macro-strain and micro-strain in the tissue site. Negative pressure can also remove exudate and other fluid from a tissue site, which can be collected in container 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

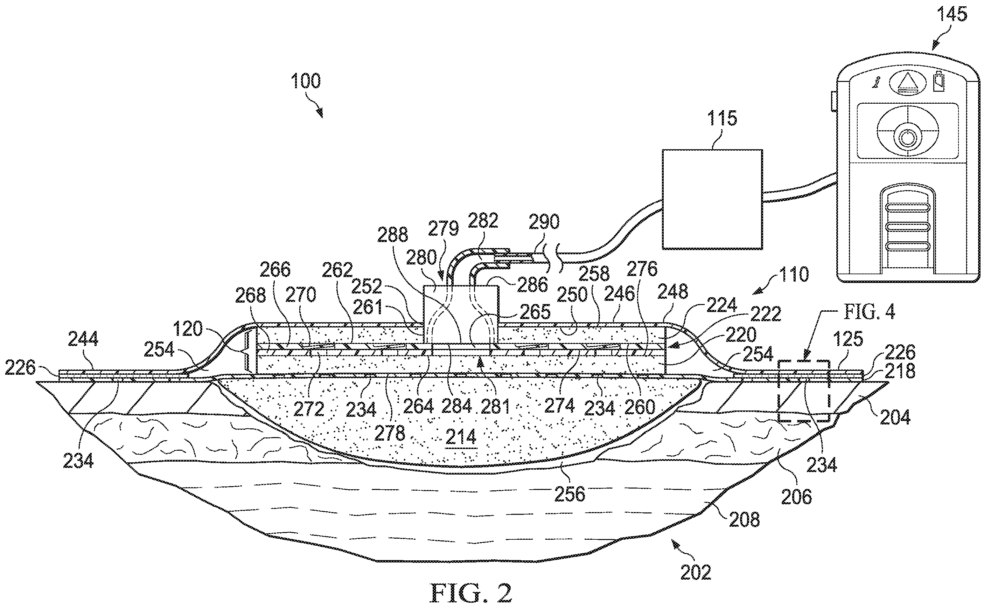

Figure 2 depicts an example embodiment of the therapy system 100 for treating a tissue site 202 of a patient. The tissue site 202 may extend through or otherwise involve an epidermis 204, a dermis 206, and a subcutaneous tissue 208. The tissue site 202 may be a sub-surface tissue site as depicted in Figure 2 that extends below the surface of the epidermis 204. Further, the tissue site 202 may be a surface tissue site (not shown) that predominantly resides on the surface of the epidermis 204, such as, for example, an incision. The therapy system 100 may provide therapy to, for example, the epidermis 204, the dermis 206, and the subcutaneous tissue 208, regardless of the positioning of the therapy system 100 or the type of tissue site. The therapy system 100 may also be utilized without limitation at other tissue sites.

Further, the tissue site 202 may be the bodily tissue of any human, animal, or other organism, including bone tissue, adipose tissue, muscle tissue, dermal tissue, vascular tissue, connective tissue, cartilage, tendons, ligaments, or any other tissue. Treatment of the tissue site 202 may include removal of fluids, e.g., exudate or ascites.

Continuing with Figure 2, the therapy system 100 may include the dressing 110, the container 115, and the therapy unit 145 that may include the negative-pressure source 105. Further, the therapy system 100 may include a filler material 214 as an optional component of the therapy system 100 that may be omitted for different types of tissue sites or different types of therapy using negative pressure, such as, for example, epithelialization. If equipped, the filler material 214 may be adapted to be positioned proximate to or adjacent to the tissue site 202, such as, for example, by cutting or otherwise shaping the filler material 214 in any suitable manner to fit the tissue site 202 and to fill a space between the tissue site 202 and the dressing 110. Similar to the tissue interface 120, the filler material 214 may be constructed of the manifold materials described herein and may be adapted to be positioned in fluid communication with the tissue site 202 to distribute negative pressure to the tissue site 202. In some embodiments, the filler material 214 may be positioned in direct contact with the tissue site 202 and between the tissue site 202 and the dressing 110. If the filler material 214 is omitted, the tissue interface 120 of the dressing 110 may be positioned in direct contact with the tissue site 202.

Continuing with Figure 2, the dressing 110 may be adapted to provide or distribute negative pressure from the negative-pressure source 105 of the therapy unit 145 to the tissue site 202 directly or through the filler material 214, if equipped. Further, Figure 2 illustrates additional features that may be associated with some example embodiments of the tissue interface 120 of the dressing 110. For example, the tissue interface 120 of the dressing 110 may include an optional base layer 218, a manifold 220, an isolation layer 222, and an absorbent material 224. An adhesive layer such as an adhesive 226 may be configured to be positioned between the cover 125 and a periphery of the tissue site 202 to secure the dressing 110 relative to the tissue site 202. Components of the dressing 110 may be added or removed to suit a particular application.

Referring to Figures 2-6, the base layer 218 may have a periphery 230 surrounding a central portion 232, and a plurality of apertures 234 disposed through the periphery 230 and the central portion 232. The base layer 218 may also have corners 236 and edges 238. The corners 236 and the edges 238 may be part of the periphery 230. One of the edges 238 may meet another of the edges 238 to define one of the corners 236. Further, the base layer 218 may have a border 240 substantially surrounding the central portion 232 and positioned between the central portion 232 and the periphery 230. The border 240 may be free of the apertures 234. The base layer 218 may cover the tissue site 202 and tissue surrounding the tissue site 202 such that the central portion 232 of the base layer 218 is positioned adjacent to or proximate to the tissue site 202, and the periphery 230 of the base layer 218 is positioned adjacent to or proximate to tissue surrounding the tissue site 202. In this manner, the periphery 230 of the base layer 218 may surround the tissue site 202. Further, the apertures 234 in the base layer 218 may be in fluid communication with the tissue site 202 and tissue surrounding the tissue site 202.

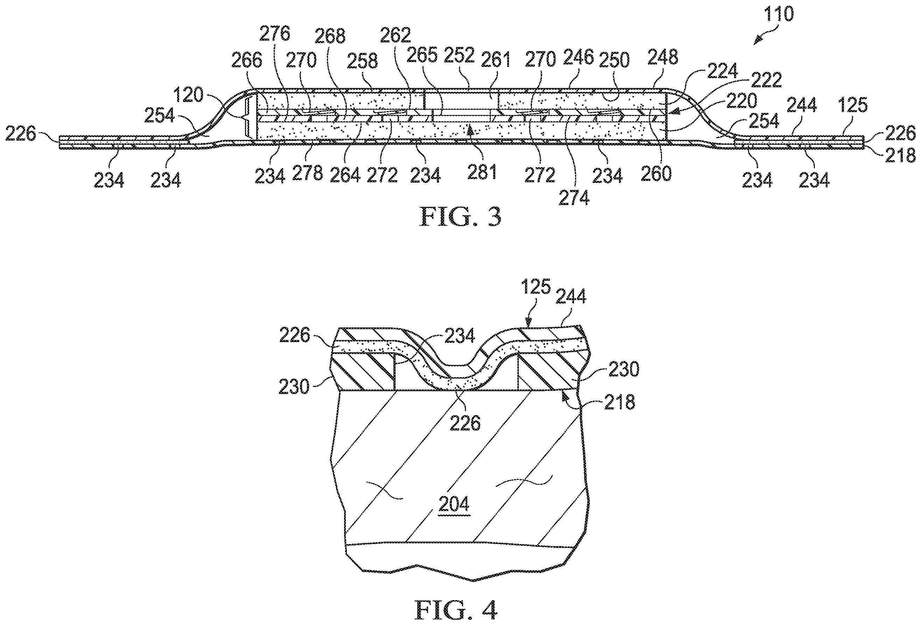

The apertures 234 in the base layer 218 may have any shape, such as, for example, circles, squares, stars, ovals, polygons, slits, complex curves, rectilinear shapes, triangles, or other shapes. The apertures 234 may be formed by cutting, by application of local RF energy, or other suitable techniques for forming an opening. As shown in Figures 4-6, each of the apertures 234 of the plurality of apertures 234 may be substantially circular in shape, having a diameter and an area. The area of each of the apertures 234 may refer to an open space or open area defining each of the apertures 234. The diameter of each of the apertures 234 may define the area of each of the apertures 234. For example, the area of one of the apertures 234 may be defined by multiplying the square of half the diameter of the aperture 234 by the value 3.14. Thus, the following equation may define the area of one of the apertures 234: Area = 3.14*(diameter/2)^2. The area of the apertures 234 described in the illustrative embodiments herein may be substantially similar to the area in other embodiments (not shown) for the apertures 234 that may have non-circular shapes. The diameter of each of the apertures 234 may be substantially the same, or each of the diameters may vary depending, for example, on the position of the aperture 234 in the base layer 218. For example, the diameter of the apertures 234 in the periphery 230 of the base layer 218 may be larger than the diameter of the apertures 234 in the central portion 232 of the base layer 218. Further, the diameter of each of the apertures 234 may be between about 1 millimeter to about 50 millimeters. In some embodiments, the diameter of each of the apertures 234 may be between about 1 millimeter to about 20 millimeters. The apertures 234 may have a uniform pattern or may be randomly distributed on the base layer 218. The size and configuration of the apertures 234 may be designed to control the adherence of the dressing 110 to the epidermis 204 as described below.

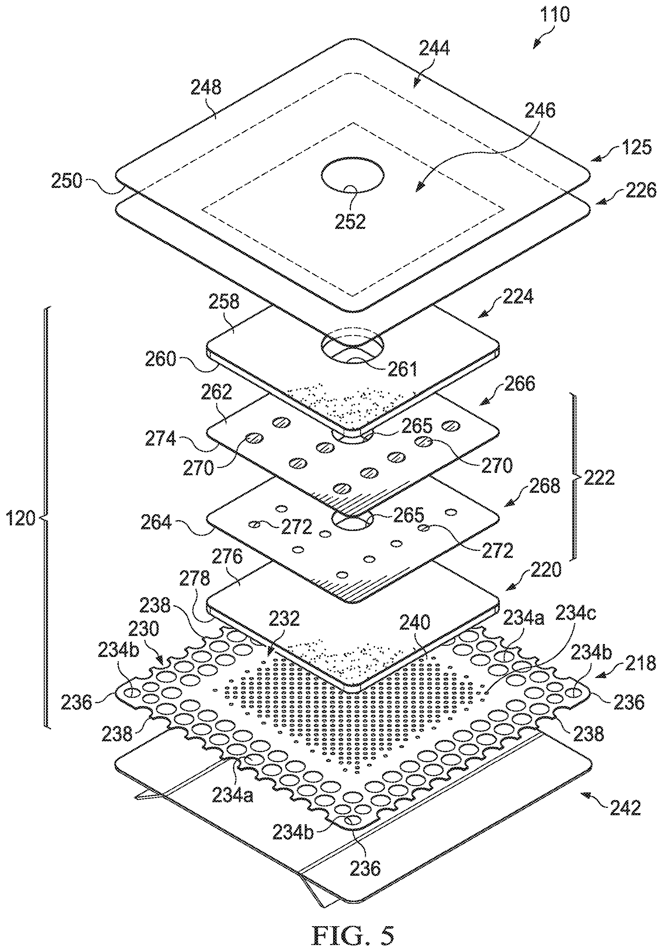

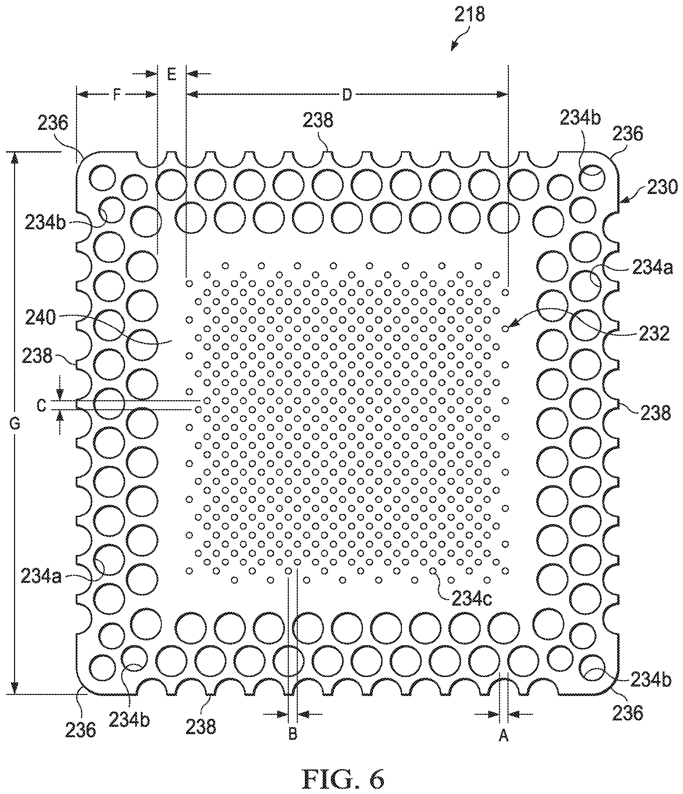

Referring to Figure 5 and 6, in some embodiments, the apertures 234 positioned in the periphery 230 may be apertures 234a, the apertures 234 positioned at the corners 236 of the periphery 230 may be apertures 234b, and the apertures 234 positioned in the central portion 232 may be apertures 234c. The apertures 234a may have a diameter between about 9.8 millimeters to about 10.2 millimeters. The apertures 234b may have a diameter between about 7.75 millimeters to about 8.75 millimeters. The apertures 234c may have a diameter between about 1.8 millimeters to about 2.2 millimeters. The diameter of each of the apertures 234a may be separated from one another by a distance A between about 2.8 millimeters to about 3.2 millimeters. Further, the diameter of at least one of the apertures 234a may be separated from the diameter of at least one of the apertures 234b by the distance A. The diameter of each of the apertures 234b may also be separated from one another by the distance A. A center of one of the apertures 234c may be separated from a center of another of the apertures 234c in a first direction by a distance B between about 2.8 millimeters to about 3.2 millimeters. In a second direction transverse to the first direction, the center of one of the apertures 234c may be separated from the center of another of the apertures 234c by a distance C between about 2.8 millimeters to about 3.2 millimeters. As shown in Figures 5-6, the distance B and the distance C may be increased for the apertures 234c in the central portion 232 being positioned proximate to or at the border 240 compared to the apertures 234c positioned away from the border 240.

As shown in Figures 5-6, the central portion 232 of the base layer 218 may be substantially square with each side of the central portion 232 having a length D between about 100 millimeters to about 108 millimeters. In some embodiments, the length D may be between about 106 millimeters to about 108 millimeters. The border 240 of the base layer 218 may have a width E between about 4 millimeters to about 11 millimeters and may substantially surround the central portion 232 and the apertures 234c in the central portion 232. In some embodiments, the width E may be between about 9 millimeters to about 10 millimeters. The periphery 230 of the base layer 218 may have a width F between about 25 millimeters to about 35 millimeters and may substantially surround the border 240 and the central portion 232. In some embodiments, the width F may be between about 26 millimeters to about 28 millimeters. Further, the periphery 230 may have a substantially square exterior with each side of the exterior having a length G between about 154 millimeters to about 200 millimeters. In some embodiments, the length G may be between about 176 millimeters to about 184 millimeters. Although Figures 5-6 depict the central portion 232, the border 240, and the periphery 230 of the base layer 218 as having a substantially square shape, these and other components of the base layer 218 may have any shape to suit a particular application. Further, the dimensions of the base layer 218 as described herein may be increased or decreased, for example, substantially in proportion to one another to suit a particular application. The use of the dimensions in the proportions described above may enhance the cosmetic appearance of a tissue site. For example, these proportions may provide a surface area for the base layer 218, regardless of shape, that is sufficiently smooth to enhance the movement and proliferation of epithelial cells at the tissue site 202, and reduce the likelihood of granulation tissue in-growth into the dressing 110.

The base layer 218 may be a soft, pliable material suitable for providing a fluid seal with the tissue site 202 as described herein. For example, the base layer 218 may comprise a silicone gel, a soft silicone, hydrocolloid, hydrogel, polyurethane gel, polyolefin gel, hydrogenated styrenic copolymer gels, a foamed gel, a soft closed cell foam such as polyurethanes and polyolefins coated with an adhesive described below, polyurethane, polyolefin, or hydrogenated styrenic copolymers. The base layer 218 may have a thickness between about 500 microns (µm) and about 1000 microns (µm). In some embodiments, the base layer 218 has a stiffness between about 5 Shore OO and about 80 Shore OO. The base layer 218 may be comprised of hydrophobic or hydrophilic materials.

In some embodiments (not shown), the base layer 218 may be a hydrophobic-coated material. For example, the base layer 218 may be formed by coating a spaced material, such as, for example, woven, nonwoven, molded, or extruded mesh with a hydrophobic material. The hydrophobic material for the coating may be a soft silicone, for example. In this manner, the adhesive 226 may extend through openings in the spaced material analogous to the apertures 234 described below.

The adhesive 226 may be in fluid communication with the apertures 234 in at least the periphery 230 of the base layer 218. In this manner, the adhesive 226 may be in fluid communication with the tissue surrounding the tissue site 202 through the apertures 234 in the base layer 218. As described below and shown in FIG. 4, the adhesive 226 may extend or be pressed through the plurality of apertures 234 to contact the epidermis 204 for securing the dressing 110 to, for example, the tissue surrounding the tissue site 202. The apertures 234 may provide sufficient contact of the adhesive 226 to the epidermis 204 to secure the dressing 110 about the tissue site 202. However, the configuration of the apertures 234 and the adhesive 226, described below, may permit release and repositioning of the dressing 110 about the tissue site 202.

At least one of the apertures 234a in the periphery 230 of the base layer 218 may be positioned at the edges 238 of the periphery 230 and may have an interior cut open or exposed at the edges 238 that is in fluid communication in a lateral direction with the edges 238. The lateral direction may refer to a direction toward the edges 238 and in the same plane as the base layer 218. As shown in Figures 5-6, a plurality of the apertures 234a in the periphery 230 may be positioned proximate to or at the edges 238 and in fluid communication in a lateral direction with the edges 238. The apertures 234a positioned proximate to or at the edges 238 may be spaced substantially equidistant around the periphery 230 as shown in Figures 5-6. However, in some embodiments, the spacing of the apertures 234a proximate to or at the edges 238 may be irregular. The adhesive 226 may be in fluid communication with the edges 238 through the apertures 234a being exposed at the edges 238. In this manner, the apertures 234a at the edges 238 may permit the adhesive 226 to flow around the edges 238 for enhancing the adhesion of the edges 238 around the tissue site 202, for example.

Continuing with Figures 5-6, the apertures 234b at the corners 236 of the periphery 230 may be smaller than the apertures 234a in other portions of the periphery 230 as described above. For a given geometry of the corners 236, the smaller size of the apertures 234b compared to the apertures 234a may maximize the surface area of the adhesive 226 exposed and in fluid communication through the apertures 234b at the corners 236. For example, as shown in Figures 5-6, the edges 238 may intersect at substantially a right angle, or about 90 degrees, to define the corners 236. Also as shown, the corners 236 may have a radius of about 10 millimeters. Three of the apertures 234b having a diameter between about 7.75 millimeters to about 8.75 millimeters may be positioned in a triangular configuration at the corners 236 to maximize the exposed surface area for the adhesive 226. The size and number of the apertures 234b in the corners 236 may be adjusted as necessary, depending on the chosen geometry of the corners 236, to maximize the exposed surface area of the adhesive 226 as described above. Further, the apertures 234b at the corners 236 may be fully housed within the base layer 218, substantially precluding fluid communication in a lateral direction exterior to the corners 236. The apertures 234b at the corners 236 being fully housed within the base layer 218 may substantially preclude fluid communication of the adhesive 226 exterior to the corners 236, and may provide improved handling of the dressing 110 during deployment at the tissue site 202. Further, the exterior of the corners 236 being substantially free of the adhesive 226 may increase the flexibility of the corners 236 to enhance comfort.

Similar to the apertures 234b in the corners 236, any of the apertures 234 may be adjusted in size and number to maximize the surface area of the adhesive 226 in fluid communication through the apertures 234 for a particular application or geometry of the base layer 218. For example, in some embodiments (not shown) the apertures 234b, or apertures of another size, may be positioned in the periphery 230 and at the border 240. Similarly, the apertures 234b, or apertures of another size, may be positioned as described above in other locations of the base layer 218 that may have a complex geometry or shape.

The adhesive 226 may be a medically-acceptable adhesive. The adhesive 226 may also be flowable. For example, the adhesive 226 may comprise an acrylic adhesive, rubber adhesive, high-tack silicone adhesive, polyurethane, or other adhesive substance. In some embodiments, the adhesive 226 may be a pressure-sensitive adhesive comprising an acrylic adhesive with coating weight of 15 grams/m² (gsm) to 70 grams/m² (gsm). The adhesive 226 may be a layer having substantially the same shape as the periphery 230 of the base layer 218 as shown in Figure 5. In some embodiments, the layer of the adhesive 226 may be continuous or discontinuous. Discontinuities in the adhesive 226 may be provided by apertures (not shown) in the adhesive 226. The apertures in the adhesive 226 may be formed after application of the adhesive 226 or by coating the adhesive 226 in patterns on a carrier layer, such as, for example, a side of the cover 125 adapted to face the epidermis 204. Further, the apertures in the adhesive 226 may be sized to control the amount of the adhesive 226 extending through the apertures 234 in the base layer 218 to reach the epidermis 204. The apertures in the adhesive 226 may also be sized to enhance the Moisture Vapor Transfer Rate (MVTR) of the dressing 110.

Factors that may be utilized to control the adhesion strength of the dressing 110 may include the diameter and number of the apertures 234 in the base layer 218, the thickness of the base layer 218, the thickness and amount of the adhesive 226, and the tackiness of the adhesive 226. An increase in the amount of the adhesive 226 extending through the apertures 234 generally corresponds to an increase in the adhesion strength of the dressing 110. A decrease in the thickness of the base layer 218 generally corresponds to an increase in the amount of adhesive 226 extending through the apertures 234. Thus, the diameter and configuration of the apertures 234, the thickness of the base layer 218, and the amount and tackiness of the adhesive utilized may be varied to provide a desired adhesion strength for the dressing 110. For example, the thickness of the base layer 218 may be about 200 microns, the layer of adhesive 226 may have a thickness of about 30 microns and a tackiness of 2000 grams per 25 centimeter wide strip, and the diameter of the apertures 234a in the base layer 218 may be about 10 millimeters.

In some embodiments, the tackiness of the adhesive 226 may vary in different locations of the base layer 218. For example, in locations of the base layer 218 where the apertures 234 are comparatively large, such as the apertures 234a, the adhesive 226 may have a lower tackiness than other locations of the base layer 218 where the apertures 234 are smaller, such as the apertures 234b and 234c. In this manner, locations of the base layer 218 having larger apertures 234 and adhesive 226 with lower tackiness may have an adhesion strength comparable to locations having smaller apertures 234 and adhesive 226 with higher tackiness.

Clinical studies have shown that the configuration described herein for the base layer 218 and the adhesive 226 may reduce the occurrence of blistering, erythema, and leakage when in use. Such a configuration may provide, for example, increased patient comfort and increased durability of the dressing 110.

Referring to the embodiment of Figure 5, a release liner 242 may be attached to or positioned adjacent to the base layer 218 to protect the adhesive 226 prior to application of the dressing 110 to the tissue site 202. Prior to application of the dressing 110 to the tissue site 202, the base layer 218 may be positioned between the cover 125 and the release liner 242. Removal of the release liner 242 may expose the base layer 218 and the adhesive 226 for application of the dressing 110 to the tissue site 202. The release liner 242 may also provide stiffness to assist with, for example, deployment of the dressing 110. The release liner 242 may be, for example, a casting paper, a film, or polyethylene. Further, the release liner 242 may be a polyester material such as polyethylene terephthalate (PET), or similar polar semi-crystalline polymer. The use of a polar semi-crystalline polymer for the release liner 242 may substantially preclude wrinkling or other deformation of the dressing 110. For example, the polar semi-crystalline polymer may be highly orientated and resistant to softening, swelling, or other deformation that may occur when brought into contact with components of the dressing 110, or when subjected to temperature or environmental variations, or sterilization. Further, a release agent may be disposed on a side of the release liner 242 that is configured to contact the base layer 218. For example, the release agent may be a silicone coating and may have a release factor suitable to facilitate removal of the release liner 242 by hand and without damaging or deforming the dressing 110. In some embodiments, the release agent may be flourosilicone. In other embodiments, the release liner 242 may be uncoated or otherwise used without a release agent.

Continuing with Figures 2-6, the cover 125 may be substantially as described above with reference to Figure 1. The cover 125 may have a margin or a periphery 244 and a central portion 246. The cover 125 may also have a first surface 248 and a second surface 250 opposite the first surface 248. The cover 125 may additionally include an aperture 252. The aperture 252 may be an opening or a hole through the cover 125. In some embodiments, the aperture 252 may be located substantially centered on the cover 125. The aperture 252 may be configured to allow fluid communication from the first surface 248 of the cover 125 through the dressing 110. The periphery 244 of the cover 125 may be positioned proximate to the periphery 230 of the base layer 218 such that the central portion 246 of the cover 125 and the central portion 232 of the base layer 218 define an enclosure 254.

The adhesive 226 may be positioned at least between the periphery 244 of the cover 125 and the periphery 230 of the base layer 218. The cover 125 may cover the tissue site 202 and the tissue interface 120 to provide a fluid seal and a sealed space 256 between the tissue site 202 and the cover 125 of the dressing 110. Further, the cover 125 may cover other tissue, such as a portion of the epidermis 204, surrounding the tissue site 202 to provide the fluid seal between the cover 125 and the tissue site 202. In some embodiments, a portion of the periphery 244 of the cover 125 may extend beyond the periphery 230 of the base layer 218 and into direct contact with tissue surrounding the tissue site 202. In other embodiments, the periphery 244 of the cover 125, for example, may be positioned in contact with tissue surrounding the tissue site 202 to provide the sealed space 256 without the base layer 218. Thus, the adhesive 226 may also be positioned at least between the periphery 244 of the cover 125 and tissue, such as the epidermis 204, surrounding the tissue site 202. The adhesive 226 may be disposed on a surface of the cover 125 adapted to face the tissue site 202 and the base layer 218.

The absorbent material 224, the isolation layer 222 and the manifold 220 may be disposed within the enclosure 254, the sealed space 256, or both. The isolation layer 222 may be positioned between the absorbent material 224 and the manifold 220, the absorbent material 224 may be encapsulated between the cover 125 and the isolation layer 222, and the manifold 220 may be positioned between the isolation layer 222 and the tissue site 202 and the base layer 218, if equipped.

The absorbent material 224 may be a super absorbent and may have a first surface 258 and a second surface 260 opposite the first surface 258. The absorbent material 224 may further include an opening 261 configured to align with the aperture 252 of the cover 125. The first surface 258 of the absorbent material 224 may be adjacent to the second surface 250 of the cover 125. According to the invention, the absorbent material 224 is isolated from the manifold 220 and the tissue site 202 when negative pressure is being applied to the tissue site 202. When negative pressure is not being applied to the tissue site 202, the absorbent material 224 is in fluid communication with the manifold 220 and the tissue site 202 through the isolation layer 222. The absorbent material 224 may be configured to absorb fluid from the tissue site 202 when the absorbent material 224 is in fluid communication with the manifold 220 and the tissue site 202 through the isolation layer 222. Materials suitable for the absorbent material may include Luquafleece^{®} material, Texsus FP2326, BASF 402C, Technical Absorbents 2317 available from Technical Absorbents (www.techabsorbents.com), sodium polyacrylate super absorbers, cellulosics (carboxy methyl cellulose and salts such as sodium CMC), or alginates.

The isolation layer 222 may be disposed between the absorbent material 224 and the manifold 220. The isolation layer 222 may have a first surface 262 and a second surface 264 opposite the first surface 262. The first surface 262 of the isolation layer 222 may be adjacent to the second surface 260 of the absorbent material 224. The isolation layer 222 may further include an opening 265. The opening 265 may be aligned with the opening 261 of the absorbent material 224 but may be smaller than the opening 261 of the absorbent material 224. The isolation layer 222 may be configured to restrict fluid flow from the tissue site 202 to the absorbent material 224 when negative pressure is applied to the dressing 110.

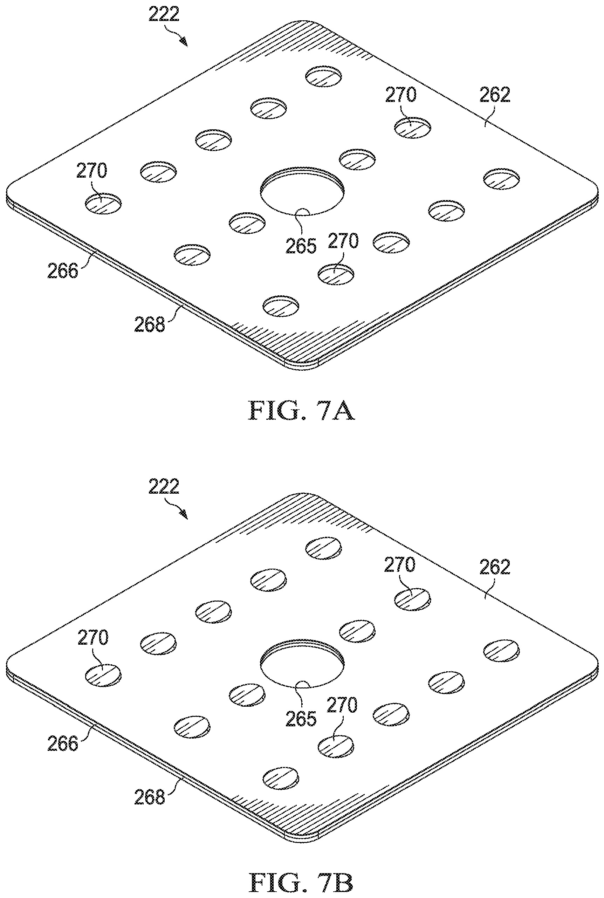

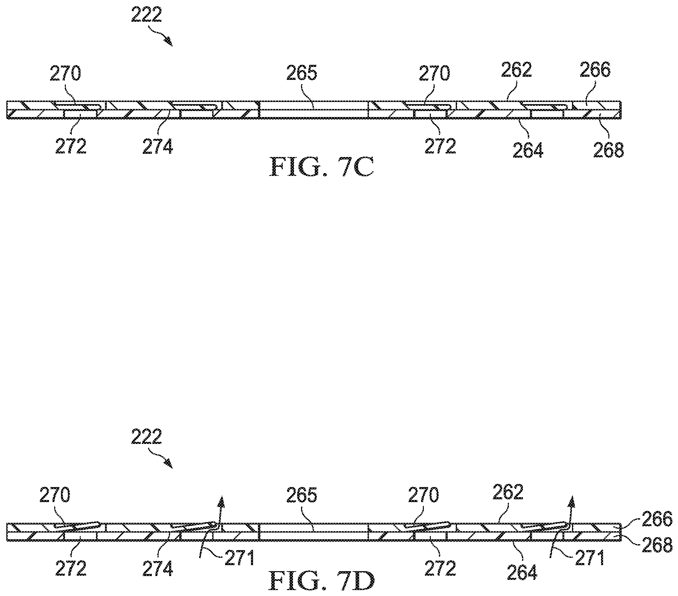

Continuing with Figures 2, 3, 5, and 7A-7D, the isolation layer 222 may include a first layer 266 and a second layer 268. The first layer 266 may include a plurality of valve flaps 270 and the second layer may include a plurality of holes 272. The plurality of holes 272 of the second layer 268 may be aligned with the plurality of valve flaps 270 of the first layer 266. Referring to Figure 7C, the plurality of valve flaps 270 may be configured to be closed when negative pressure is applied to the dressing 110. When closed, the plurality of valve flaps 270 may fluidly seal the plurality of holes 272 of the second layer 268. Referring to Figure 7D, the plurality of valve flaps 270 may be configured to move away from the plurality of holes 272 when negative pressure is not being applied to the dressing 110 to provide a passage 271 through the isolation layer 222. The plurality of valve flaps 270 may be configured to permit fluid flow in a first direction from the second surface 264 of the isolation layer 222 towards the first surface 262 of the isolation layer 222 when negative pressure is not being applied to the dressing 110. The plurality of valve flaps 270 may prevent fluid flow in a second direction from the first surface 262 of the isolation layer 222 towards the second surface 264 of the isolation layer 222. When negative pressure is being applied to the dressing 110, the plurality of valve flaps 270 may prevent fluid flow in all directions, for example, both the first direction and the second direction. In other embodiments, the isolation layer 222 may be only one layer that may include the plurality of valve flaps 270 that are configured to close when negative pressure is applied to the dressing 110 and are configured to open when negative pressure is not being applied to the dressing to provide a passage through the isolation layer 222.

The first layer 266 of the isolation layer 222 may have a first surface that may be the first surface 262 of the isolation layer 222. The first layer 266 of the isolation layer 222 may have a second surface 274 opposite the first surface 262. The plurality of valve flaps 270 may be configured to open from the second surface 274 of the first layer 266 towards the first surface 262 of the isolation layer 222. The first layer 266 of the isolation layer 222 may be thick enough to enable the plurality of valve flaps 270 to open and expose the plurality of holes 272 of the second layer 268 without any portion of the plurality of valve flaps 270 coming into contact with the absorbent material 224.

The plurality of valve flaps 270 of the isolation layer 222 may be check valves in some embodiments. Exemplary check valves may include ball check valves, diaphragm check valves, flap-style check valves, swing check valves, stop-check valves, duckbill valves, pneumatic non-return valves, or other one-way valves configured to automatically permit fluid flow in a single direction and to prevent fluid flow in any other direction.

In some embodiments, there may be at least one spacer or projection positioned between the isolation layer 222 and the absorbent material 224. The at least one spacer or projection may be positioned around or adjacent to one or more of the valve flaps 270 and configured in any suitable manner to provide a pathway or additional space between the isolation layer 222 and the absorbent material 224. The pathway may ensure that the plurality of valve flaps 270 have enough space to open to expose the plurality of holes 272 of the second layer 268 of the isolation layer 222 when the negative-pressure source 105 is stopped.

Both the first layer 266 and the second layer 268 of the isolation layer 222 may be comprised of a liquid impermeable film. In some embodiments, the first layer 266 and the second layer 268 of the isolation layer 222 may comprise one or more of the following materials: hydrophilic polyurethane; cellulosics; hydrophilic polyamides; polyvinyl alcohol; polyvinyl pyrrolidone; hydrophilic acrylics; hydrophilic silicone elastomers; an INSPIRE 2301 material from Expopack Advanced Coatings of Wrexham, United Kingdom having, for example, an MVTR (inverted cup technique) of 14400 g/m²/24 hours and a thickness of about 30 microns; a thin, uncoated polymer drape; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; polyurethane (PU); EVA film; co-polyester; silicones; a silicone drape; a 3M Tegaderm^{®} drape; a polyurethane (PU) drape such as one available from Avery Dennison Corporation of Pasadena, California; polyether block polyamide copolymer (PEBAX), for example, from Arkema, France; Expopack 2327; or other appropriate material.

In some embodiments, the first layer 266 and the second layer 268 of the isolation layer 222 may be a flexible, breathable film, membrane, or sheet having a high MVTR of, for example, at least about 300g/m² per 24 hours. In other embodiments, a low or no vapor transfer drape might be used. The first layer 266 and the second layer 268 of the isolation layer 222 may comprise a range of medically suitable films having a thickness between about 15 microns (µm) to about 50 microns (µm). In other embodiments, the first layer 266 and the second layer 268 of the isolation layer 222 may be a non-breathable film, membrane, or sheet that may be substantially vapor and liquid impermeable.

Continuing with Figures 2, 3, and 5, the manifold 220 may have a first surface 276 and a second surface 278 opposite the first surface 276. The manifold 220 may comprise or consist essentially of a means for distributing fluid relative to the tissue site 202. For example, the manifold 220 may be adapted to receive negative pressure from the negative-pressure source 105 and distribute negative pressure through multiple apertures across or through the manifold 220, which may have the effect of collecting fluid from the tissue site 202 and drawing the fluid toward the negative-pressure source 105.

In some illustrative embodiments, the manifold 220 may comprise a plurality of pathways, which can be interconnected to improve distribution or collection of fluids. In some illustrative embodiments, the manifold 220 may comprise or consist essentially of a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, the manifold 220 may additionally or alternatively comprise projections that form interconnected fluid pathways. For example, the manifold 220 may be molded to provide surface projections that define interconnected fluid pathways.

In some embodiments, the manifold 220 may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) may be particularly suitable for some types of therapy. The tensile strength of the manifold 220 may also vary according to needs of a prescribed therapy. The 25% compression load deflection of the manifold 220 may be at least 0.35 pounds per square inch, and the 65% compression load deflection may be at least 0.43 pounds per square inch. In some embodiments, the tensile strength of the manifold 220 may be at least 10 pounds per square inch. The manifold 220 may have a tear strength of at least 2.5 pounds per inch. In some embodiments, the manifold 220 may be foam comprised of polyols such as polyester or polyether, isocyanate such as toluene diisocyanate, and polymerization modifiers such as amines and tin compounds. In some examples, the manifold 220 may be reticulated polyurethane foam such as found in GRANUFOAM^{™} dressing or V.A.C. VERAFLO^{™} dressing, both available from Kinetic Concepts, Inc. of San Antonio, Texas.

The thickness of the manifold 220 may also vary according to needs of a prescribed therapy. For example, the thickness of the manifold 220 may be decreased to reduce tension on peripheral tissue of the tissue site 202. The thickness of the manifold 220 can also affect the conformability of the manifold 220. In some embodiments, a thickness in a range of about 5 millimeters to 10 millimeters may be suitable.

In some exemplary embodiments, the manifold 220 may be hydrophilic. In an example in which the manifold 220 may be hydrophilic, the manifold 220 may also wick fluid away from a tissue site, while continuing to distribute negative pressure to the tissue site 202. The wicking properties of the manifold 220 may draw fluid away from the tissue site 202 by capillary flow or other wicking mechanisms. An example of a hydrophilic material that may be suitable is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

Continuing with Figure 2, with reference to Figure 3, a conduit interface 279 may be configured to fluidly couple the dressing 110 to the container 115 and the therapy unit 145. The conduit interface 279 may include a fluid connection 280 and a negative pressure port 282. The negative pressure port 282 may be disposed through the fluid connection 280 and the fluid connection 280 may fluidly isolate the negative pressure port 282 from the absorbent material 224. The fluid connection 280 may extend into or be received by a negative pressure passage 281 defined by an alignment of the aperture 252 of the cover 125, the opening 261 of the absorbent material 224, and the opening 265 in the isolation layer 222. The fluid connection 280 may have a first surface 284 that may be configured to couple to the first surface 262 of the isolation layer 222. The fluid connection 280 may have a second surface 286 opposite the first surface 284. In other embodiments, the fluid connection 280 may extend through the cover 125, the absorbent material 224, and the isolation layer 222 to couple to the first surface 276 of the manifold 220.

The negative pressure port 282 may extend through the fluid connection 280 from the second surface 286 to the first surface 284. The negative pressure port 282 may have an end 288 that may be configured to deliver negative pressure from the negative-pressure source 105 to the manifold 220. The end 288 of the negative pressure port 282 may be proximate to the opening 265 of the isolation layer 222 and below or between the second surface 260 of the absorbent material 224 and the manifold 220 to deliver negative pressure through the opening 265 to the manifold 220. In some examples, the end 288 of the negative pressure port 282 may be fluidly sealed about the opening 265 of the isolation layer 222. In other examples, the negative pressure port 282 may bypass or extend through the absorbent material 224 such that the end 288 is positioned at or between the second surface 260 of the absorbent material 224 and the manifold 220. The negative pressure port 282 may couple to a conduit 290 that may couple to the container 115 and the negative-pressure source 105 of the therapy unit 145.

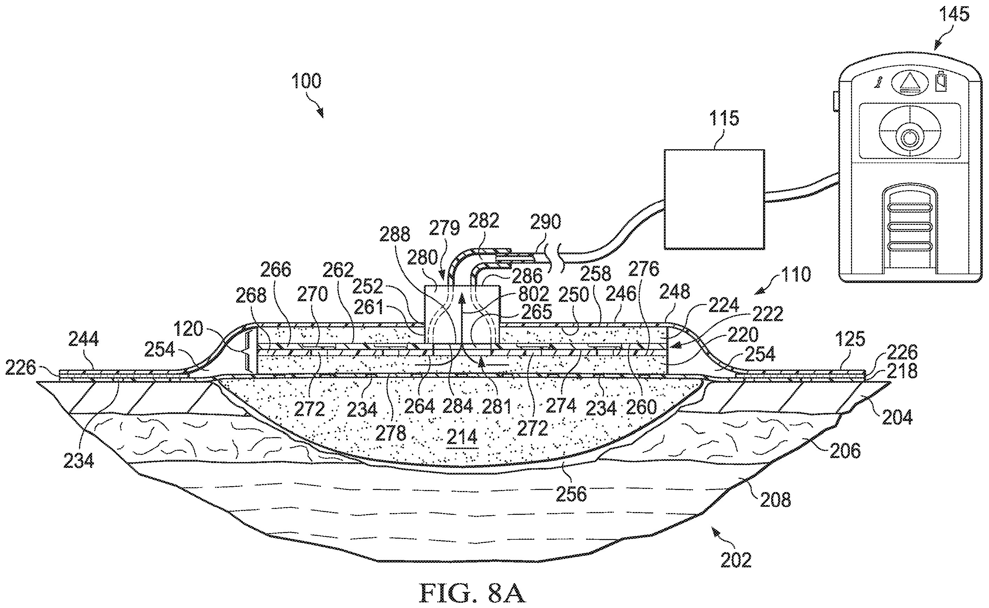

Figure 8A depicts the therapy system 100 of Figure 2 in an operational state with the negative-pressure source 105 of the therapy unit 145 delivering negative pressure to the tissue site 202. In operation, the negative-pressure source 105 may draw fluid or exudate 802 from the tissue site 202 and the exudate 802 may flow through the optional filler material 214, the optional base layer 218, and the manifold 220 to reach the negative pressure passage 281. The exudate 802 may then flow through the negative pressure passage 281 to the negative pressure port 282 and through the conduit 290 to the container 115. The container 115 may have filters or other mechanisms to retain the exudate 802 within the container 115 and prevent aspiration of the exudate 802 into the therapy unit 145.

The plurality of valve flaps 270 of the first layer 266 of the isolation layer 222 may be closed when the negative-pressure source 105 is actuated. The plurality of valve flaps 270 may fluidly seal the plurality of holes 272 of the second layer 268 of the isolation layer 222. While the negative-pressure source 105 is actuated, the negative pressure passage 281 may be in direct fluid communication with the manifold 220 and fluidly isolated from the absorbent material 224 such that the exudate 802 may not reach the absorbent material 224.

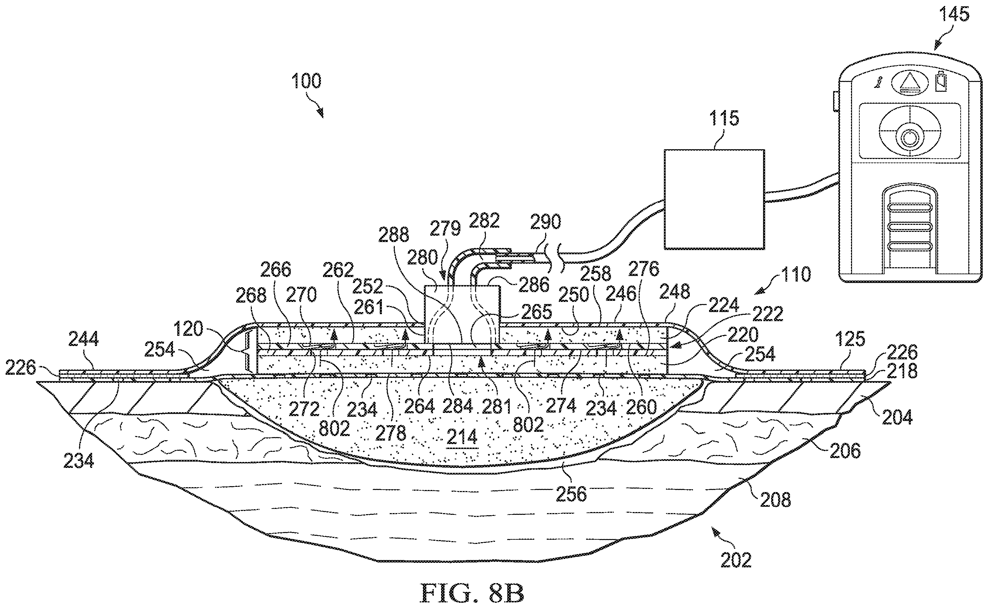

Figure 8B depicts the therapy system 100 of Figure 2 and 8A after the negative-pressure source 105 has stopped delivering negative pressure to the tissue site 202. Once the negative-pressure source 105 has stopped delivering negative pressure to the tissue site 202, the plurality of valve flaps 270 of the first layer 266 of the isolation layer 222 may open. When open, the plurality of valve flaps 270 may expose the plurality of holes 272 of the second layer 268 of the isolation layer 222. The exudate 802 may flow from the tissue site 202 and the manifold 220 through the plurality of holes 272 and the plurality of valve flaps 270 of the isolation layer 222 and may be absorbed by the absorbent material 224. If the negative-pressure source 105 were actuated, the plurality of valve flaps 270 would close and the exudate 802 would no longer be able to reach the absorbent material 224. The exudate 802 would then flow through the negative pressure passage 281 to the container 115 of the therapy system 100.

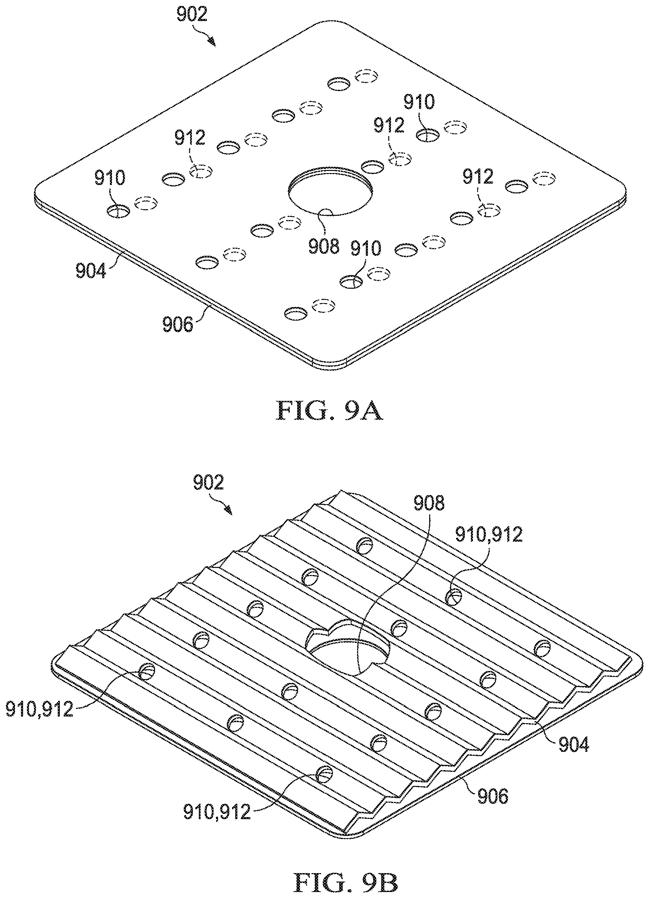

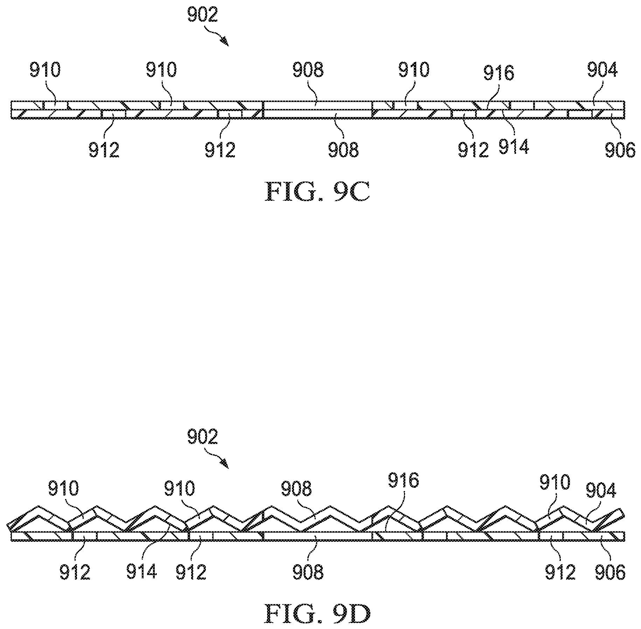

Figures 9A-9D depict another embodiment of an isolation layer 902 that can be used with the therapy system 100. The isolation layer 902 may include a first layer 904 and a second layer 906. There may be an opening 908 that extends through both the first layer 904 and the second layer 906. The first layer 904 may include a plurality of holes 910 and the second layer may include a plurality of holes 912. The first layer 904 may be corrugated or textured in a resting state as shown in Figures 9B and 9D. When in a resting state, the plurality of holes 910 of the first layer 904 and the plurality of holes 912 of the second layer 906 may allow fluid flow through the isolation layer 902.

Referring to Figures 9A and 9C, when negative pressure is applied to the dressing 110, the first layer 904 may flatten such that a second surface 914 of the first layer 904 is pressed against a first surface 916 of the second layer 906. When the first layer 904 is flat, the plurality of holes 910 of the first layer 904 may be misaligned with the plurality of holes 912 of the second layer 906 such that the plurality of holes 912 of the second layer are fluidly sealed by the first layer 904 and the plurality of holes 910 of the first layer 904 are fluidly sealed by the second layer 906. Thus, when the negative-pressure source 105 is actuated, the absorbent material 224 may be fluidly isolated from the manifold 220 and the tissue site 202. When the negative-pressure source 105 is stopped, the first layer 904 may move back to its resting state and fluid may flow through the plurality of holes 910 of the first layer 904 and the plurality of holes 912 of the second layer 906 to reach the absorbent material 224.

As described above, some embodiments of the dressing 110 may include the at least one spacer or projection to provide a pathway or space between the isolation layer 902 and the absorbent material 224. The pathway or space may allow the first layer 904 of the isolation layer 902 to move between its resting state and its flattened state when the negative-pressure source 105 is applying negative pressure to the dressing 110.

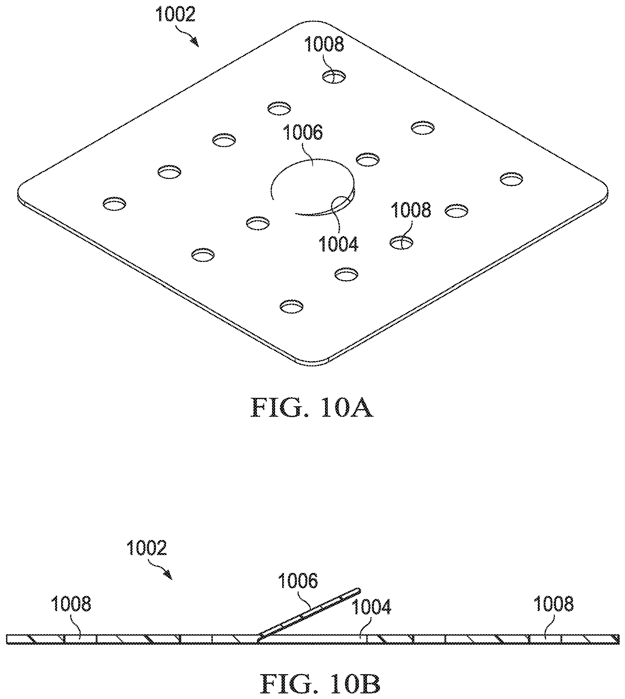

Figures 10A-10B depict another embodiment of an isolation layer 1002 that can be used with the therapy system 100. The isolation layer 1002 may include an opening 1004, a valve flap 1006, and a plurality of holes 1008 surrounding the valve flap 1006. The opening 1004 may be coupled to the negative pressure passage 281 as previously described, and the valve flap 1006 may be configured to open and expose the opening 1004 when the negative-pressure source 105 is actuated. When the negative-pressure source 105 is stopped, the valve flap 1006 may close to stop fluid communication between the dressing 110 and the negative pressure passage.

The plurality of holes 1008 may enable fluid communication between the absorbent material 224 and the manifold 220. However, when the negative-pressure source 105 is actuated, the exudate 802 may flow preferentially from the tissue site 202 through the manifold 220, the opening 1004, and through the negative pressure passage 281 to the container 115. Some of the exudate 802 may flow through the plurality of holes 1008 and be absorbed by the absorbent material 224 but most of the exudate 802 will be pulled into the container 115 by the negative-pressure source 105. When the negative-pressure source 105 is stopped, the valve flap 1006 may close the opening 1004 and the exudate 802 may flow through the plurality of holes 1008 of the isolation layer 1002 and be absorbed by the absorbent material 224.

In some embodiments, the plurality of holes 1008 may each have a corresponding valve flap to restrict fluid flow through the isolation layer 1002 when the negative-pressure source 105 is actuated. In any of the above described embodiments, the valve flap 1006 may be any kind of check valve that permits fluid flow in a first direction from the tissue site towards the cover 125 of the dressing 110 and restricts fluid flow in a second direction from the absorbent material 224 towards the tissue site 202.

Also described herein is a method of treating a tissue site. The method may include applying the dressing 110 to the tissue site 202, fluidly coupling the negative-pressure source 105 to a treatment space, for example, the sealed space 256 shown in Figure 2, between the isolation layer 222 and the tissue site 202 such that the absorbent material 224 is bypassed, and actuating the negative-pressure source 105 to apply negative pressure to the treatment space. The isolation layer 222 may have one or more valves, such as the plurality of valve flaps 270, which may be moved from an open state without negative pressure to a closed state by operation of the negative pressure being applied.

The method may further include stopping the negative-pressure source 105 and collecting fluids from the tissue site 202 with the absorbent material 224. The fluids may be configured to flow through the isolation layer 222 to the absorbent material 224 when the negative-pressure source 105 is stopped and the one or more valves return to the open state.

The systems, apparatuses, and methods described herein may provide significant advantages. For example, the therapy system 100 may prevent fluid from leaking from the dressing 110 after the negative-pressure source 105 is stopped. The isolation layer 222 may enable the absorbent material 224 to capture any fluid flowing from the tissue site 202 after the negative-pressure source 105 is stopped. The therapy system 100 may also prevent occlusion because the absorbent material 224 is excluded from the negative pressure pathway. This may allow the therapy system 100 to stay in place at a tissue site for longer periods of time, which may reduce costs and increase patient comfort.

## Claims

1. A dressing for treating a tissue site with negative pressure, the dressing comprising:
a cover (125) comprising a first surface and a second surface opposite the first surface;
an absorbent material (224) comprising a first surface and a second surface opposite the first surface, the first surface of the absorbent material (224) adjacent to the second surface of the cover (125);
an isolation layer (222, 902) configured to restrict fluid flow from the tissue site to the absorbent material (224) when a negative pressure is applied to the dressing, the isolation layer (222, 902) comprising a first surface and a second surface opposite the first surface, the first surface of the isolation layer (222, 902) adjacent to the second surface of the absorbent material (224); and
a manifold (220) comprising a first surface and a second surface opposite the first surface, the first surface of the manifold (220) adjacent to the second surface of the isolation layer (222, 902);
wherein each of the cover (125), the absorbent material (224), and the isolation layer (222, 902) further comprise a negative pressure passage aligned (281) with each other and configured to enable fluid communication between the manifold (220) and a negative-pressure source (145); and
wherein the negative pressure passage (281) is in direct fluid communication with the manifold (220) and fluidly isolated from the absorbent material (224).

2. The dressing of claim 1, wherein the isolation layer is positioned between the absorbent material (224) and the manifold (220).

3. The dressing of claim 1, wherein the absorbent material (224) is encapsulated between the cover (125) and the isolation layer (222, 902).

4. The dressing of claim 1, wherein the manifold (220) is configured to be positioned between the isolation layer (222, 902) and the tissue site.

5. The dressing of claim 1, wherein the isolation layer (222, 902) comprises one or more valves (270) configured to close when the negative pressure is applied and to open when the negative pressure is removed.

6. The dressing of claim 5, wherein the one or more valves are check valves configured to permit fluid flow in a first direction from the second surface of the isolation layer (222, 902) toward the first surface of the isolation layer (222, 902) and to prevent fluid flow in a second direction from the first surface of the isolation layer (222, 902) toward the second surface of the isolation layer (222, 902).

7. The dressing of claim 5, wherein the one or more valves prevent fluid flow in all directions when the negative pressure is being applied.

8. The dressing of claim 5, wherein each of the one or more valves includes a valve flap (270) configured to close a hole through the isolation layer (222, 902) when the negative pressure is applied and to move away from the hole to provide a passage through the isolation layer (222, 902) when the negative pressure is removed.

9. The dressing of claim 5, wherein the isolation layer (222, 902) comprises a liquid impermeable film.

10. The dressing of claim 1, wherein the isolation layer (222) comprises:
a first layer (266) comprising a plurality of valve flaps (270); and
a second layer (268) comprising a plurality of holes (272) configured to align with the plurality of valve flaps (270) of the first layer (266) and to be fluidly sealed by the plurality of valve flaps (270) of the first layer (266) when the negative pressure is applied to the dressing.

11. The dressing of claim 1, wherein the isolation layer comprises:
a first layer (904) comprising a corrugated material with a first plurality of holes (910); and
a second layer (906) comprising a second plurality of holes (912) configured to be fluidly sealed by the first layer (904) when the negative pressure is applied to the dressing.

12. The dressing of claim 11, wherein the first plurality of holes (910) and the second plurality of holes (912) are configured to provide fluid communication between the manifold (220) and the absorbent material (224) when the dressing is free of the negative pressure in a resting state, and wherein the first plurality of holes (910) are configured to be misaligned with the second plurality of holes (912) when the negative pressure is applied to the dressing to prevent fluid communication between the manifold (220) and the absorbent material (224).

13. The dressing of claim 1, wherein the negative pressure passage (281) is configured to receive a fluid connection (280), and wherein the fluid connection (280) includes a negative pressure port (282) positioned between the second surface of the absorbent material (224) and the manifold (224), wherein optionally the fluid connection fluidly isolates the negative pressure port from the absorbent material (224).

14. The dressing of claim 1, wherein the cover (125) comprises a margin with an adhesive layer that extends beyond the absorbent material (224), the isolation layer (222), and the manifold (220).

15. The dressing of claim 14, further comprising a base layer (218) adjacent to the second surface of the manifold (220) and configured to extend into contact with the margin of the cover (125) to enclose the absorbent material (224), the isolation layer (222), and the manifold (220) between the base layer (218) and the cover (125), wherein the base layer (218) comprises a silicone gel having a plurality of apertures.

## Patentansprüche

1. Ein Verband zum Behandeln einer Gewebestelle mit Unterdruck, der Verband aufweisend:
eine Abdeckung (125), aufweisend eine erste Oberfläche und einer der ersten Oberfläche gegenüberliegende zweite Oberfläche;
ein absorbierendes Material (224), aufweisend eine erste Oberfläche und eine der ersten Oberfläche gegenüberliegende zweite Oberfläche, wobei die erste Oberfläche des absorbierenden Materials (224) an die zweite Oberfläche der Abdeckung (125) angrenzt;
eine Isolierschicht (222, 902), die konfiguriert ist, um einen Fluidfluss von der Gewebestelle zu dem absorbierenden Material (224) zu beschränken, wenn auf den Verband ein Unterdruck angelegt wird, die Isolierschicht (222, 902) aufweisend eine erste Oberfläche und eine der ersten Oberfläche gegenüberliegende zweite Oberfläche, wobei die erste Oberfläche der Isolierschicht (222, 902) an die zweite Oberfläche des absorbierenden Materials (224) angrenzt; und
einen Verteiler (220), aufweisend eine erste Oberfläche und eine der ersten Oberfläche gegenüberliegende zweite Oberfläche, wobei die erste Oberfläche des Verteilers (220) an die zweite Oberfläche der Isolierschicht (222, 902) angrenzt;
wobei jedes von der Abdeckung (125), des absorbierenden Materials (224) und der Isolierschicht (222, 902) ferner einen Unterdruckdurchgang aufweisen, der aneinander ausgerichtet (281) und konfiguriert ist, um eine Fluidkommunikation zwischen dem Verteiler (220) und einer Unterdruckquelle (145) zu ermöglichen; und
wobei der Unterdruckdurchgang (281) in direkter Fluidkommunikation mit dem Verteiler (220) steht und von dem absorbierenden Material (224) fluidisch isoliert ist.

2. Der Verband nach Anspruch 1, wobei die Isolierschicht zwischen dem absorbierenden Material (224) und dem Verteiler (220) angeordnet ist.

3. Der Verband nach Anspruch 1, wobei das absorbierende Material (224) zwischen der Abdeckung (125) und der Isolierschicht (222, 902) eingekapselt ist.

4. Der Verband nach Anspruch 1, wobei der Verteiler (220) konfiguriert ist, um zwischen der Isolierschicht (222, 902) und der Gewebestelle positioniert zu werden.

5. Der Verband nach Anspruch 1, wobei die Isolierschicht (222, 902) ein oder mehrere Ventile (270) aufweist, die konfiguriert sind, um sich zu schließen, wenn der Unterdruck angelegt wird, und sich öffnen, wenn der Unterdruck entfernt wird.

6. Der Verband nach Anspruch 5, wobei das eine oder die mehreren Ventile Rückschlagventile sind, die konfiguriert sind, um einen Fluidfluss in einer ersten Richtung von der zweiten Oberfläche der Isolierschicht (222, 902) zu der ersten Oberfläche der Isolierschicht (222, 902) hin zuzulassen und um einen Fluidfluss in einer zweiten Richtung von der ersten Oberfläche der Isolierschicht (222, 902) zu der zweiten Oberfläche der Isolierschicht (222, 902) hin zu verhindern.

7. Der Verband nach Anspruch 5, wobei das eine oder die mehreren Ventile den Fluidfluss in alle Richtungen verhindern, wenn der Unterdruck angelegt wird.

8. Der Verband nach Anspruch 5, wobei jedes des einen oder der mehreren Ventile eine Ventilklappe (270) enthält, die konfiguriert ist, um ein Loch über die Isolierschicht (222, 902) zu schließen, wenn der Unterdruck angelegt wird, und um sich von dem Loch weg zu bewegen, um einen Durchgang über die Isolierschicht (222, 902) bereitzustellen, wenn der Unterdruck entfernt wird.

9. Der Verband nach Anspruch 5, wobei die Isolierschicht (222, 902) einen flüssigkeitsundurchlässigen Film aufweist.

10. Der Verband nach Anspruch 1, wobei die Isolierschicht (222) aufweist:
eine erste Schicht (266), aufweisend eine Mehrzahl von Ventilklappen (270); und
eine zweite Schicht (268), aufweisend eine Mehrzahl von Löchern (272), die konfiguriert ist, um an der Mehrzahl von Ventilklappen (270) der ersten Schicht (266) ausgerichtet zu sein und um durch die Mehrzahl von Ventilklappen (270) der ersten Schicht (266) fluiddicht verschlossen zu werden, wenn der Unterdruck auf den Verband angelegt wird.

11. Der Verband nach Anspruch 1, wobei die Isolierschicht aufweist:
eine erste Schicht (904), aufweisend ein geripptes Material mit einer Mehrzahl von Löchern (910); und
eine zweite Schicht (906), aufweisend eine zweite Mehrzahl von Löchern (912), die konfiguriert ist, um durch die erste Schicht (904) fluiddicht verschlossen zu werden, wenn der Unterdruck auf den Verband angelegt wird.

12. Der Verband nach Anspruch 11, wobei die erste Mehrzahl von Löchern (910) und die zweite Mehrzahl von Löchern (912) konfiguriert sind, um die Fluidkommunikation zwischen dem Verteiler (220) und dem absorbierenden Material (224) bereitzustellen, wenn der Verband in einem Ruhezustand frei von Unterdruck ist, und wobei die erste Mehrzahl von Löchern (910) konfiguriert ist, um nicht an der zweiten Mehrzahl von Löchern (912) ausgerichtet zu sein, wenn der Unterdruck auf den Verband angelegt wird, um die Fluidkommunikation zwischen dem Verteiler (220) und dem absorbierenden Material (224) zu verhindern.

13. Der Verband nach Anspruch 1, wobei der Unterdruckdurchgang (281) konfiguriert ist, um eine Fluidkommunikation (280) aufzunehmen, und wobei die Fluidkommunikation (280) einen Unterdruckanschluss (282) enthält, der zwischen der zweiten Oberfläche des absorbierenden Materials (224) und dem Verteiler (224) positioniert ist, wobei optional die Fluidkommunikation den Unterdruckanschluss von dem absorbierenden Material (224) fluidisch isoliert.

14. Der Verband nach Anspruch 1, wobei die Abdeckung (125) einen Rand mit einer Klebeschicht aufweist, die sich über das absorbierende Material (224), die Isolierschicht (222) und den Verteiler (220) hinaus erstreckt.

15. Der Verband nach Anspruch 14, ferner aufweisend eine Basisschicht (218) angrenzend an die zweite Oberfläche des Verteilers (220), und die konfiguriert ist, um sich in Kontakt mit dem Rand der Abdeckung (125) zu erstrecken, um das absorbierende Material (224), die Isolierschicht (222) und den Verteiler (220) zwischen der Basisschicht (218) und der Abdeckung (125) einzuschließen, wobei die Basisschicht (218) ein Silikongel aufweist, die eine Mehrzahl von Öffnungen aufweist.

## Revendications

1. Pansement permettant de traiter un site tissulaire par pression négative, le pansement comprenant :
une couverture (125) comprenant une première surface et une seconde surface opposée à la première surface ;
un matériau absorbant (224) comprenant une première surface et une seconde surface opposée à la première surface, la première surface du matériau absorbant (224) étant adjacente à la seconde surface de la couverture (125) ;
une couche d'isolation (222, 902) conçue pour restreindre un écoulement de fluide allant du site tissulaire au matériau absorbant (224) lorsqu'une pression négative est appliquée au pansement, la couche d'isolation (222, 902) comprenant une première surface et une seconde surface opposée à la première surface, la première surface de la couche d'isolation (222, 902) étant adjacente à la seconde surface du matériau absorbant (224) ; et
un collecteur (220) comprenant une première surface et une seconde surface opposée à la première surface, la première surface du collecteur (220) étant adjacente à la seconde surface de la couche d'isolation (222, 902) ;
dans lequel chacun parmi la couverture (125), le matériau absorbant (224) et la couche d'isolation (222, 902) comprend en outre un passage de pression négative aligné (281) les uns avec les autres et configuré pour permettre une communication fluidique entre le collecteur (220) et une source de pression négative (145) ; et
dans lequel le passage de pression négative (281) est en communication fluidique directe avec le collecteur (220) et isolé fluidiquement du matériau absorbant (224).

2. Pansement selon la revendication 1, dans lequel la couche d'isolation est positionnée entre le matériau absorbant (224) et le collecteur (220).

3. Pansement selon la revendication 1, dans lequel le matériau absorbant (224) est encapsulé entre la couverture (125) et la couche d'isolation (222, 902).

4. Pansement selon la revendication 1, dans lequel le collecteur (220) est conçu pour être positionné entre la couche d'isolation (222, 902) et le site tissulaire.

5. Pansement selon la revendication 1, dans lequel la couche d'isolation (222, 902) comprend une ou plusieurs valves (270) conçues pour se fermer lorsque la pression négative est appliquée et pour s'ouvrir lorsque la pression négative est retirée.

6. Pansement selon la revendication 5, dans lequel la ou les valves sont valves antiretour conçues pour permettre un écoulement de fluide dans une première direction allant de la seconde surface de la couche d'isolation (222, 902) à la première surface de la couche d'isolation (222, 902) et pour empêcher un écoulement de fluide dans une seconde direction allant de la première surface de la couche d'isolation (222, 902) à la seconde surface de la couche d'isolation (222, 902).

7. Pansement selon la revendication 5, dans lequel la ou les valves empêchent un écoulement de fluide dans toutes les directions lorsque la pression négative est en cours d'application.

8. Pansement selon la revendication 5, dans lequel chacune parmi la ou les valves comporte un clapet de valve (270) conçu pour fermer un trou à travers la couche d'isolation (222, 902) lorsque la pression négative est appliquée et pour s'éloigner du trou pour fournir un passage à travers la couche d'isolation (222, 902) lorsque la pression négative est retirée.

9. Pansement selon la revendication 5, dans lequel la couche d'isolation (222, 902) comprend un film imperméable aux liquides.

10. Pansement selon la revendication 1, dans lequel la couche d'isolation (222) comprend :
une première couche (266) comprenant une pluralité de clapets de valve (270) ; et
une seconde couche (268) comprenant une pluralité de trous (272) conçus pour s'aligner avec la pluralité de clapets de valve (270) de la première couche (266) et pour être isolés fluidiquement par la pluralité de clapets de valve (270) de la première couche (266) lorsque la pression négative est appliquée au pansement.

11. Pansement selon la revendication 1, dans lequel la couche d'isolation comprend :
une première couche (904) comprenant un matériau ondulé avec une première pluralité de trous (910) ; et
une seconde couche (906) comprenant une seconde pluralité de trous (912) conçus pour être isolés fluidiquement par la première couche (904) lorsque la pression négative est appliquée au pansement.

12. Pansement selon la revendication 11, dans lequel la première pluralité de trous (910) et la seconde pluralité de trous (912) sont conçus pour fournir une communication fluidique entre le collecteur (220) et le matériau absorbant (224) lorsque le pansement est dépourvu de pression négative dans un état au repos, et dans lequel la première pluralité de trous (910) sont conçus pour être désalignés par rapport à la seconde pluralité de trous (912) lorsque la pression négative est appliquée au pansement pour empêcher une communication fluidique entre le collecteur (220) et le matériau absorbant (224).

13. Pansement selon la revendication 1, dans lequel le passage de pression négative (281) est conçu pour recevoir un raccordement fluidique (280), et dans lequel le raccordement fluidique (280) comporte un orifice de pression négative (282) positionné entre la seconde surface du matériau absorbant (224) et le collecteur (224), dans lequel facultativement le raccordement fluidique isole fluidiquement l'orifice de pression négative du matériau absorbant (224).

14. Pansement selon la revendication 1, dans lequel la couverture (125) comprend une bordure avec une couche adhésive qui s'étend au-delà du matériau absorbant (224), de la couche d'isolation (222) et du collecteur (220).

15. Pansement selon la revendication 14, comprenant en outre une couche de base (218) adjacente à la seconde surface du collecteur (220) et conçue pour s'étendre en contact avec la bordure de la couverture (125) pour enfermer le matériau absorbant (224), la couche d'isolation (222) et le collecteur (220) entre la couche de base (218) et la couverture (125), dans lequel la couche de base (218) comprend un gel de silicone ayant une pluralité d'ouvertures.
